(19) Europäisches Patentamt
European Patent Office
Office européen des brevets

(11) Publication number: **0 256 682 A2**

(12) # EUROPEAN PATENT APPLICATION

(21) Application number: 87306464.6

(51) Int. Cl.⁴: **A01N 63/00 , C12N 15/00**

(22) Date of filing: 22.07.87

(30) Priority: 04.08.86 GB 8619008

(43) Date of publication of application:
24.02.88 Bulletin 88/08

(84) Designated Contracting States:
AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Shaw, Charles H.
15 Crossview Terrace
Neville's Cross Durham(GB)
Inventor: Ashby, Alison M.
Pear Tree Cottage The Green
Old Cornforth County Durham(GB)
Inventor: Richards, Andrew J. M.
Pear Tree Cottage The Green
Old Cornforth County Durham(GB)
Inventor: Watson, Martin D.
5 Mitford Close, High Shincliffe
Durham(GB)

(74) Representative: Roberts, Timothy Wace et al
Imperial Chemical Industries PLC Legal
Department : Patents PO Box 6 Bessemer
Road
Welwyn Garden City Herts, AL7 1HD(GB)

(54) **Plant treatment.**

(57) A method of delivering a pesticidal material to a plant which comprises applying to the plant, or to the environment adjacent the plant, a microorganism capable of exhibiting a chemotactic response to a plant metabolite, whereby movement of said microorganism towards the plant as a result of chemotaxis results in delivery or production of pesticidal material at or adjacent the plant.

Fig. 4.

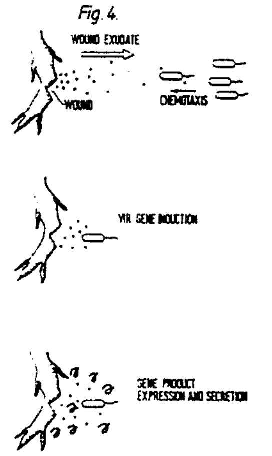


EP 0 256 682 A2

Xerox Copy Centre

## PLANT TREATMENT

This invention relates to a method of treating plants and particularly but not exclusively to a method of protecting plants against insect and fungal attack by applying thereto substances active pesticidally, eg. as fungicides and insecticides.

Conventional methods of applying pesticides to plants suffer the disadvantage that precise application of pesticide to a desired location is impractical and consequently pesticides are typically applied in a wide spread and haphazard manner by for example spraying. The result often is that more pesticide has to be introduced into the environment than would be necessary if the pesticide could be applied accurately to the location of the plant or released only in the event of attack of individual plants by pests.

The present invention is based on the discovery that certain microorganisms exhibit a chemotactic response to plant metabolites and can be adapted to mediate in the release or production of a treatment substance at or near the location of the plant. The production of treatment substance in accordance with the invention can avoid the inefficiency of previous methods, since the production of treatment substance occurs generally only when required and at a desired location. The method of the invention in its widest aspects involves applying such microorganisms to or adjacent to the plants to be treated.

Thus according to a first aspect of the invention there is provided a method of delivering a pesticidal material to a plant which comprises applying to the plant, or to the environment adjacent the plant, a microorganism capable of exhibiting a chemotactic response to a plant metabolite, whereby movement of said microorganism towards the plant as a result of chemotaxis results in delivery or production of pesticidal material at or adjacent the plant. The term "metabolite" as used herein is intended to encompass not only the products of normal metabolism, but also products formed during breakdown of plant tissue as a result of cell death, pathenogenesis or wounding.

The chemical species to which the microorganism exhibits a chemotactic response may, for example, be a metabolite released by the plant in response to wounding, for example wounding caused mechanically or wounding caused by insect or fungal attack, in which case the microorganism will tend to be drawn towards the wounded plants and effect delivery or production of the desired pesticidal material adjacent to the plants, specifically in the area of the wound.

The presence of the microorganisms may effect delivery or production of the pesticidal material in various ways. Thus, for example, in a preferred embodiment of the invention, the pesticidal material is a product of expression of one or more chromosomal or non-chromosomal genes, preferably a gene constructed by recombinant DNA techniques and used to transform the microorganism used in the method of the invention.

Conveniently, the gene or genes which are expressed to produce the pesticidal material may be induced by the same plant metabolite to which the microorganism exhibits a chemotactic response.

Thus the plant metabolite to which the microorganism exhibits a chemotactic response may also act as an inducer of the gene or genes which are expressed to produce the pesticidal material, in which case not only will the microorganism be drawn towards the plants as a result of chemotaxis, but the presence of the afore-mentioned plant metabolite (which is likely to be present in a relatively high concentration closer to individual plants) will also induce the production of the pesticidal material.

Transformed microorganisms for use in carrying out the methods described are themselves novel. Thus according to a further aspect of the invention there are provided transformed microorganisms capable of exhibiting a chemotactic response to a plant metabolite and including an inducible chromosomal or non-chromosomal gene coding for a pesticidally active polypeptide.

The invention also visualises plasmids useful in transformation to produce such microorganisms which comprise a replication site, a gene induction sequence adapted to be activated by a plant metabolite, and a gene for production of a pesticidally active polypeptide under control of said gene induction sequence; and novel DNA base sequences found in such plasmids.

In accordance with a further aspect of the invention which is not necessarily dependent upon the use of a microorganism capable of exhibiting a chemotactic response, a pesticidal material may be delivered to a plant by introducing into the environment a microorganism capable of producing the pesticidal material directly or indirectly by expression of one or more chromosomal or non-chromosomal genes and inducing the expression of said genes by applying an inducer of said gene or genes to or adjacent to the plant to be treated.

The invention is particularly applicable to combat diseases and pests of plant roots, for example nematodes, corn root worm etc.

In carrying out the method of the invention, the microorganisms may retain latent in the soil and can become activated to produce pesticidal material in response to wounding or pest attack. Alternatively the microorganisms can be activated by applying an inducer as described above.

Examples of pesticidal material which may be delivered or produced in accordance with the invention include pesticidally active enzymes such as, for example, chitinases, glucanases and mannanases capable of digesting fungal cell walls or other components of fungi and insects, for example the chitinase of Serratia marcescens.

A further class of pesticidally active substances which can be delivered or produced in accordance with the invention are insecticidal proteins including the deltaendotoxin of Bacillus thuringiensis.

A further class of pesticidally active substances which can be delivered or produced in accordance with the invention are enzyme inhibitors such as trypsin and $\alpha$-amylase inhibitors.

A further class of pesticidal materials which may be delivered or produced in accordance with the invention are insect neuropeptides or peptide hormones, or inhibitory analogues of such peptides.

A further class of pesticidal materials which may be delivered or produced in accordance with the invention are substances which remove from the environment essential elements or growth factors necessary for fungal or insecticidal growth. Examples include siderophores which when produced in or delivered to the environment adjacent to a plant can prevent the growth of pathogens by chelating metal ions necesary for the growth of pathogens. Further examples of materials which act in this way include avidin and streptavidin which cause a depletion of biotin in the environment of the plant, thus inhibiting the growth of biotin-dependent pathogens.

A further mechanism whereby pesticidal material may be delivered or produced following movement of a microorganism towards a plant in accordance with the invention is where the microorganism triggers the plant to produce phytoalexins or other known insecticidal plant metabolites.

Alternatively, the microorganism species may be capable of producing an agent capable of assisting the healing of a plant wound, eg. plant growth regulators or cellulose, in which case the cellulose produced by the microorganism in the locality of the plant wound can act to plug the wound. Thus according to a further aspect of the invention there is provided a method of promoting healing of a plant wound which comprises applying to a plant or to the environment adjacent the plant a microorganism capable of exhibiting a chemotactic response to a plant metabolite and of producing an agent capable of assisting the healing of a plant wound, whereby movement of said microorganism towards the plant as a result of chemotaxis results in delivery to or production of said agent at the wound site.

Examples of plant metabolites to which the microorganism used in accordance with the method of the invention can exhibit a chemotactic response include :

acetosyringone
alpha hydroxyacetosyringone
3,4-dihydroxybenzoic acid (protocatechoic acid)
catechol
vanilylalcohol
3,4-dihydroxybenzaldehyde
ferulic acid
traumatic acid
vanillic acid
gibberelic acid
indole acetic acid
vanillin
p-hydroxybenzoic acid
beta-resorcyclic acid
pyrogallic acid
gallic acid
luteolin
apigenin
sugars

The method of the invention is not limited to use of any particular microorganism, but particularly encouraging results have been obtained with Agrobacterium species, particularly Agrobacterium tumefaciens. Agrobacterium species are naturally occuring in the soil environment and are thus suited to survive in the soil. Particularly we have found that the strain C58C[1] (pTiB6S3) which harbours the octopine Ti plasmid pTiB6S3 exhibits positive chemotaxis towards the phenolic plant wound exudate acetosyringone.

It has previously been reported that the ability of strains of Agrobacterium tumefaciens to induce tumours on dicotyledonous plants results from the transfer of a small segment of DNA termed T-DNA. The process is dependent on a 40 kb sector called the virulence or vir-region which is situated to the left (ie. the upstream or 5′ side) of the T-DNA on the tumour-inducing (Ti) plasmid. Plant wounding initiates a complex series of actions involving vir-gene induction, T-DNA transfer to plant cells and eventual tumour formation.

Wounded plant tissue exudes a conglomerate of chemicals both aromatic and aliphatic. Amongst the aromatics released are acetosyringone (4-acetyl-2,6-dimethoxyphenol (AS), alpha-hydroxyacetosyringone (4-(2-hydroxyacetyl)-2,6-dimethoxyphenol) and vanilyl alcohol, all of which occur as biosynthetic precursors or breakdown products of lignin. AS is a phenolic compound of Mw = 196 and chemical composition $C_{10}H_{12}O_4$ (Figure 1) and vanilyl alcohol is a phenolic compound of Mw = 154 and chemical composition $C_8H_{10}O_3$ (Figure 1). Included amongst the aliphatic compounds are wound hormones such as traumatic acid (trans-2-dodecenedioic acid) and indoleacetic acid (IAA).

Preferably strains of A. tumefaciens used in accordance with the invention harbor variants of the Ti plasmid which are avirulent, that is to say the strains used exhibit a chemotactic response but are incapable of inducing the so-called crown gall symptoms in the plants being treated. Such plasmids are also termed "dis-armed".

It has surprisingly been found that strains of the species A. tumefaciens harbouring a Ti plasmid exhibit a chemotactic response at concentrations of plant metabolite attractant as low as $10^{-7}$ molar. This is two orders of magnitude lower than the levels of such metabolites which have been found to induce the vir genes.

It is thus a further feature of the invention to utilise as the selected microorganism which is applied to the plant or the environment adjacent the plant, a strain of microorganism which harbours a recombinant plasmid formed by introducing into the Ti plasmid of A. tumefaciens, a DNA segment coding for a desired gene product. Preferably such DNA segment is under regulatory control of one or more vir genes, whereby expression of the gene product may be dependent upon activation of the vir gene. The Ti plasmid itself is preferably disarmed.

It will be appreciated that by constructing such a recombinant plasmid, microorganisms may be produced which when used in accordance with the invention exhibit a chemotactic effect at a relatively low concentration of a plant metabolite and move towards a plant which is producing the metabolite, thus encountering a higher concentration whereby the vir gene will be induced and as a result expression of the gene product will occur. The selected microorganism which harbours the aforementioned plasmid may be A. tumefaciens itself or another bacterial species (preferably a Gram-negative bacterial species, eg. Pseudomonas or Rhizobium) capable of being transformed with the Ti plasmid.

For bacterial species which are not under natural conditions capable of supporting replication of the Ti plasmid, operation in accordance with the invention may be faciliated by constructing a broad host range replicon including a recognition site for a plant metabolite, a vir gene, and one or more genes for production of pesticidal material under control of the vir gene.

The invention will be further described with reference to the drawings, in which :

Figure 1 shows the chemical formulae of two chemotaxy-inducing plant metabolites;

Figure 2 illustrates a broad host range plasmid;

Figure 3 illustrates how a gene fragment coding for chitinase can be placed under the control of a vir gene to give DNA base sequences according to the invention;

Figure 4 shows the movement of microorganisms according to the invention towards wounded plants, followed by the induction of the pesticide-encoding gene in the higher concentration of plant metabolites adjacent the wounded plant.

Figures 5 and 6 show how chemotaxy towards acetosyringone and vanilyl alcohol differs as between Ti-and Ti + plasmids.

Figure 7 shows a scheme for making a vir B cassette.

Figure 8 shows a scheme for making a chitinase cassette.

Figure 9 shows the production of a plasmid according to the invention from vir B and chitinase cassettes.

Figure 10 shows the production of a second plasmid useful in microorganisms according to the invention.

As can be seen from the following experimental results, it has been demonstrated that A. tumefaciens exhibits a specific chemotactic response to acetosyringone which is a Ti plasmid determined function, although chemotaxis per se is not Ti plasmid encoded.

Experimental Protocol

I. Organisms and Media

The strains used were A. tumefaciens C58C[1] (pTiB6S3) (De Greve, H Decraemer, H Seurinck, J Van Montagu, M and Schell, J (I98I) Plasmid 6, 325-248; C58C' (pDVBI003Δ3I) (Shaw C H, Watson M D, Carter G H and Shaw C H (I984) Nucl Acids Res I2, 603I-604I); C58C[1] (Van Larebeke, NEngler, G Holsters, M Van den Elsacker, S Zaenen, I Schilperoort, R A and Schell, J (I974) Nature 252, I69-I70) and E. coli MCI022 (Casadabhan,M J and Cohen, S N (I980) J Molec.Biol. I38, I79-207). Strain C58C[1] was chosen as the strain lacking a Ti plasmid and the E. coli strain was adapted to glycerol salts medium (Adler J (I973) J Gen. Microbiol. 74, 77-9I) and used as a control strain.

Chemotaxis medium (Adler J (I973) J. Gen. Microbiol. 74, 77-9I) was autoclaved before use. Acetosyringone and vanilyl alcohol (both from Aldrich) were filter sterilised.

2. Chemotaxis Assay

300μl from an overnight bacterial culture were inoculated into I0 ml of L-broth in a I00 ml flask to give an approximate absorbance of 0.05 at 590 nm. The flasks were incubated with shaking until the cells had reached exponential phase, at a culture density of approximately $I0^8$ $ml^{-1}$ ($_{590}$ of 0.2-0.3 in a I cm cuvette).

The culture was centrifuged at 3600g for ten minutes at I5°C and the pellet was washed in 5 ml of chemotaxis medium. After repeating the washing procedure twice, the final pellet was resuspended in up to I0 ml of chemotaxis medium giving a bacterial concentration of approximately $I0^{10}$ $ml^{-1}$.

A pool was formed by pipetting 0.3 ml of a bacterial suspension into the area created by laying a U-tube (bent from a melting point capillary tube and sealed at both ends) between a microscope slide and cover slip, giving a bacterial concentration of $I0^9$ in each pool. For large numbers of assays the microscope slides were replaced by a glass plate.

Solutions (3 ml) of the substances tested in chemotaxis medium ($I0^{-3}$ to $I0^{-12}$M) were drawn up into a capillary tube and the tube sealed at one end with vacuum grease. Capillaries were handled with forceps throughout the assay and were inserted (without rinsing) open end first into the pool containing the bacterial suspension. The assay was left for 60 minutes at room temperature.

After incubation the capillary was removed and its exterior washed with a thin stream of sterile distilled water from a wash bottle. The sealed end was broken and the contents diluted in I mM saline. Serial dilutions down to $I0^{-5}$ were made and 0.I ml of each dilution spread onto L-agar plates, with appropriate selection. Plates were incubated at 28°C for 48 hours (or for E. coli overnight at 37°C) and colonies were counted. Assays were performed in duplicate and averages reported.

Results

I. Chemotaxis Towards Acetosyringone

Acetosyringone (AS) was tested as an attractant in chemotactic assays performed on E. coli MCI022 and both A. tumefaciensC58C[1] (pTiB6S3), C58C' (pDUBI003Δ3I) and C58C[1] strains. Chemotaxis medium was used as a control. E. coli MCI022 showed no chemotaxis towards AS, yet was very motile towards $I0^{-3}$M L-aspartate in agreement with previous results.

As shown in Figure 5, the two Agrobacterium strains differed greatly in their chemotactic response, with C58C[1] showing very little alteration in bacterial numbers at different concentrations. However, this was not the case for the isogenic strains harbouring pTiB6S3 or pDUBI003 3I, which reproducibly gave a definite dose response indicating maximum motility at $I0^{-7}$. This concentration of AS is approximately I00 fold lower than that producing maximum induction of the vir-genes.

The absence of response towards AS displayed by E. coli and A. tumefaciens C58C[1] suggests that at least part of the recognition pathway involved in movement towards the attractant is Ti plasmid determined (Ashby et al, I987).

2. Chemotaxis Towards Vanilyl Alcohol

Vanilyl alcohol was diluted in chemotaxis medium through the concentration range $10^{-1}$ to $10^{-10}$M. Chemotaxis medium acted as control.

Both A. tumefaciens C58C[1] and the isogenic strain harboring pTiB6S3 were assayed as before and both strains experienced chemotaxis towards the compound. As shown in Figure 6, maximum chemotaxis occurred at $10^{-2}$. Thus chemotaxis is not a Ti plasmid determined function, but specificity towards acetosyringone is.

Experiments using A. tumefaciens C58C[1] (pDUB1003Δ31) with root and shoot extracts of dicotyledonous plants (tobacco, kalanchoe) and monocotyledonous plants (wheat) have shown chemotaxy in each case. The presence of a Ti plasmid gave enhanced chemotaxy towards wheat shoot extracts at low concentrations of extract.

The following Examples illustrate the construction of plasmids according to the invention, and plasmids useful in obtaining microorganisms useful in the methods of the invention.

EXAMPLE 1 : The VirB cassette

The Agrobacterium tumefaciens virB promoter region is contained on a 1.3kb HindIII fragment on the plasmid pSM30 (Stachel et al 1986). We have cloned this into the plasmid pUC19 (Figure 7) to give plasmid pDUB2510. The strategy aims to delete all of the virB coding sequences in this plasmid, leaving all of the promoter and translation initiation (Shine-Dalgarno) sequences. This is accompanied by digestion of pDUB2510 with BamHI and KpnI, the unidirectional degradation of the plasmid from the BamHI site into the coding sequences by Exonuclease III followed by treatment with Mung Bean Nuclease (Henikoff, 1984; Hoheisel and Pohl, 1986). The plasmid is then recircularised by ligation, incorporating a BamHI linker sequence. The extent of the deletions is measured by direct plasmid DNA sequencing (Guo et al, 1983; Chen and Seeburg, 1985) on the pDUB2510 derivatives and comparing the sequences with published data on the virB promoter structure (Das et al, 1986). Any residual codons that remain may be removed by repeating the process since Exonuclease III proceeds at a known uniform rate. The resultant HindIII - BamHI fragment in the correct pDUB2510 derivative forms a "cassette" that can be fused to the coding sequences of any gene, placing that gene under virB control when expressed in Agrobacterium tumefaciens.

EXAMPLE 2 : The Chitinase Cassette

Serratia marcescens chitinase genes have been cloned (Jones et al, 1986). One plasmid pCHIT1251, carries all of the chitinase gene on an EcoRI - HindIII fragment (Figure 8). By using a similar deletion strategy to that outlined above in Example 1, all of the chitinase promoter and 5-prime non-coding sequences from pCHIT1251 are removed. The deleted plasmids are recircularised incorporating a BamHI linker. The extent of the deletions is determined by plasmid sequencing. The resultant EcoRI - BamHI fragment in the correct derivative forms the chitinase "cassette" that can be expressed when placed in operative relation to any suitable promoter.

EXAMPLE 3 : The virB - chitinase construction

The HindIII - BamHI virB cassette, the EcoRI - BamHI chitinase cassette and EcoRI - HindIII cut pUC19 are ligated together, to give a resultant plasmid containing an EcoRI - HindIII fragment carrying the virB - chitinase fusion correctly oriented for expression (Figure 9). If this fusion in itself is found not to be ideal for virB controlled expression of chitinase, this may be corrected by synthesising a BamHI-ended adaptor oligonucleotide, designed from the sequence data obtained during the sequencing of the plasmid deletions. For example this adaptor may contain an in-frame initiator codon for chitinase, or more codons to ensure that a correct signal peptide for chitinase exists. The adaptor is inserted in to the single BamHI site between virB-and the chitinase coding sequences. This pUC19-virB-chitinase is then in turn ligated to pGV1106 (see below, Example 5). This is then introduced into A. tumefaciens.

EXAMPLE 4 : The Bacillus thuringiensis delta-endotoxin cassette

The virB controlled production of pesticidal proteins is a general strategy that can be applied to any protein for which a cloned gene exists. To exemplify this further the construction of a B. thuringiensis delta-endotoxin cassette is described. The endotoxin gene is available on a 4.6 kb PstI restriction fragment (Obukowicz et al, 1986). This is cloned into pKS$^+$, a plasmid specifically designed for the unidirectional deletion strategy described in Example I. Deletions are made into the endotoxin gene, removing all promoter and 5-prime non-coding sequences. The extent of the deletions is monitored by DNA sequencing and comparison with the published N-terminal amino acid sequence (Wong et al, 1983). The deleted plasmids are recircularised incorporating a BamHI linker. The resultant BamHI - PstI fragment forms the endotoxin cassette, which is then fused to the virB cassette of Example I as described in Example 3 above.

EXAMPLE 5 : Testing of Chitinase in Agrobacterium

pCHIT1251 is a plasmid with a HindIII - EcoRI fragment, in Example 2 above, carrying the chitinase gene. This plasmid has a single EcoRI site and is unable to replicate in Agrobacterium. pGVII06 (Leemans et al, 1982) is a broad host-range plasmid able to replicate in Agrobacterium, and is unrelated to the normal resident Ti-plasmid. It has a single EcoRI site. pCHIT1251 and pGVII06 have been ligated together via their respective EcoRI sites, to form plasmid pDUB2501 (Figure 10). This is a construct able to exist in Agrobacterium that carries the S. marcescens chitinase gene expressed from its normal promoter. Agrobacterium carrying pDUB2501 when plated onto Chitin agar produced zones of clearing, indicating that the chitinase gene was being expressed. A second S. marcescens chitinase gene is available on plasmid pCHIT310. By an identical strategy this plasmid has been fused to pGVII06 to produce plasmid pDUB2502.

The chitinase-producing Agrobacterium strains produced in Example 5 are avirulent. It may be desired to prevent them from acquiring virulence functions from another strain of Agrobacterium resident in the soil due to the transfer of a wild Ti plasmid. To inhibit such a transfer, the chitinase-producing Agrobacterium may also harbour a disarmed non-virulent Ti plasmid. This resident plasmid prevents entry of another (virulent) Ti plasmid by two mechanisms, incompatability ( inc) (Montoya et al, 1978) and establishment inhibition (ein) (Hooykaas et al, 1980). Both of these mechanisms acting together can significantly reduce the chance of chitinase-producing Agrobacterium becoming virulent.

## Example References


1. Ashby A M, Watson M D and Shaw C H (1987) FEMS Micro. Lett. 41 189-192.
2. Chen, E J and Seeburg, P H (1985) DNA 4, 165-170.
3. Das, A, Stachel, S, Allenza, P Montoya, A and Nester, E. (1986) Nucl. Acids Res 14, 1355-1364.
4. Guo, L H Yang, R C A and Wu, R (1983) Nucl. Acids Res. 11, 5521-5539.
5. Henikoff S, (1984) Gene 28, 351-359.
6. Hoheisel, J and Pohl, F M (1986) Nucl. Acids Res. 14, 3605-.
7. Hooykaas, P J J Dulk-Ras, H D Ooms, G and Schilperoort, R A (1980) J Bacteriol, 143, 1295-1306.
8. Jones, J D G Grady, K L Suslow, T V and Bedbrook, J R (1986) EMBO J. 5. 467-473.
9. Leemans J, Deblaere, R Willmitzer, L De Greve, H. Hernalsteens, J P Van Montagu M and Schell J (1982) EMBO J. 1, 147-152.
10. Montoya A L, Moore L W, Gordon M P and Nester E, W (1978) J.v Bacteriol 136, 909-915.
11. Obukowicz, M G, Perlak, F J Kusano-Kretzmer, K Mayer, E J and Watrud, L S (1986) Gene 45, 327-331.
12. Stachel, S E, Nester E W and Zambryski, P C (1986) Proc. Nat. Acad. Sci. USA. 83, 379-383.
13. Wong, H C, Schnepf, H E and Whitely, H R (1983), J Biol. Chem. 258, 1960-1967.

Deposit of Microorganisms and Plasmids

The following have been deposited at the National Collections of Industrial and Marine Bacteria (NCIB), Torry Research Station, PO Box 31, 135 Abbey Road, Aberdeen AB9 8DG, Scotland.

| Deposit | NCIB Reference |
| --- | --- |
| Plasmid pDUB2501 | 12507 |
| Plasmid pDUB2502 | 12508 |

## Claims

1. A method of delivering a pesticidal material to a plant which comprises applying to the plant, or to the environment adjacent the plant, a microorganism capable of exhibiting a chemotactic response to a plant metabolite, whereby movement of said microorganism towards the plant as a result of chemotaxis results in delivery or production of pesticidal material at or adjacent the plant.

2. A method according to claim 1 wherein the chemical species to which the microorganism exhibits a chemotactic response is a metabolite released by the plant in response to wounding, whereby the microorganism will tend to be drawn towards the wounded plant and effect delivery or production of the desired pesticidal material adjacent to the plant.

3. A method according to claim 1 or claim 2 wherein the pesticidal material is a product of expression of one or more chromosomal or non-chromosomal genes of a soil microorganism.

4. A method according to claim 3 wherein the gene is a gene which has been constructed by recombinant DNA techniques and used in the transformation of said microorganism.

5. A method according to claim 4 wherein the microorganism is of the genus Agrobacterium .

6. A method according to any preceding claim wherein the plant metabolite to which the microorganism exhibits a chemotactic response also acts as an inducer of the gene or genes which are expressed to produce the pesticidal material.

7. A method according to claims 5 and 6 wherein the gene expressing pesticidal material is under the control of the Ti plasmid virB regulatory sequence.

8. A method of delivering a pesticidal material to a plant which comprises applying to the plant, or to the environment adjacent the plant, a microorganism capable of producing said pesticidal material directly or indirectly by expression of one or more chromosomal or non-chromosomal genes and inducing the expression of said genes by applying an inducer of said gene or genes to or adjacent to the plant to be treated.

9. A method according to any preceding claim wherein the pesticidally active substance comprises an enzyme capable of digesting cell walls or other components of fungi or insects.

10. A method according to any preceding claim wherein the pesticidally active substance comprises the chitinase of Serratia marcescens.

11. A method according to any of claims 1 to 8 wherein the pesticidally active substance comprises an insecticidal protein.

12. A method according to claim 11 wherein the pesticidally active substance comprises the delta-endotoxin of Bacillus thuringiensis.

13. A method according to any of claims 1 to 8 wherein the pesticidally active substance is effective to remove from the environment essential elements or growth factors necessary for fungal or insect growth.

14. A method according to claim 13 wherein the pesticidally active substance comprises a siderophore which when produced in or delivered to the environment adjacent to a plant prevents the growth of pathogens by chelating metal ions necessary for the growth of pathogens.

15. A method according to claim 13 wherein the pesticidally active substance is effective to cause a depletion of biotin in the environment of the plant, thus inhibiting the growth of biotin-dependent pathogens.

16. A method according to any of claims 1 to 8 wherein the microorganism triggers the plant to produce an insecticidal plant metabolite.

17. A method of promoting healing of a plant wound which comprises applying to the plant or to the environment adjacent the plant a microorganism capable of exhibiting a chemotactic response to a plant metabolite and of producing an agent capable of assisting the healing of a plant wound, whereby movement of said microorganism towards the plant as a result of chemotaxis results in delivery or production of said agent at the wound site.

18. A method according to any preceding claim wherein the plant metabolite is selected from acetosyringone, alpha-hydroxyacetosyringone, 3,4-dihydroxy-benzoic acid (protocatechoic acid), catechol, vanilylalcohol, 3,4-dihydroxybenzaldehyde, ferulic acid, traumatic acid, vanillic acid, gibberelic acid, indole acetic acid, vanillin, p-hydroxybenzoic acid, beta-resorcyclic acid, pyrogallic acid and gallic acid.

19. A transformed microorganism capable of exhibiting a chemotactic response to a plant metabolite and including a recombinant chromosomal or non-chromosomal gene coding for a pesticidally active polypeptide.

20. A transformed microorganism according to claim 19 derived from E. coli, Agrobacterium tumefaciens, Rhizobium or Pseudomonas species.

21. A plasmid useful in transformation to produce microorganisms according to claims 19 or 20 which comprises a replication site, a gene induction sequence adapted to be activated by a plant metabolite, and a gene for production of a pesticidally active polypeptide under control of said gene induction sequence.

22. A plasmid as claimed in claim 21 wherein the gene induction sequence is the promoter and Shine-Dalgarno sequence of the virB gene of the Ti plasmid.

23. A plasmid as claimed in either of claims 21 or 22 in which the gene under control of the gene induction sequence serves to express the chitinase of Serratia marcescens or the δ-endotoxin of Bacillus thuringiensis.

24. A DNA base sequence useful in the production of genetic material such as the plasmids of claims 21-23 which comprises a gene induction sequence adapted to be activated by a plant wound metabolite and a gene for production of a pesticidally active polypeptide positioned on the 5′ side of said sequence so as to be under control thereof.

*Fig. 1.*

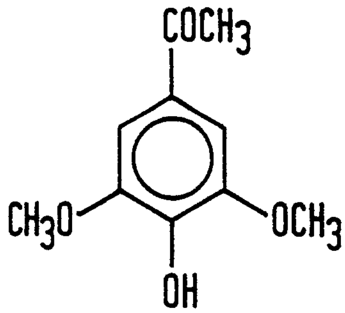

ACETOSYRINGONE ('AS')

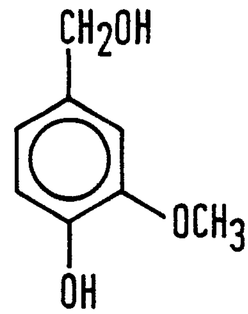

VANILYLALCOHOL

*Fig. 2.*

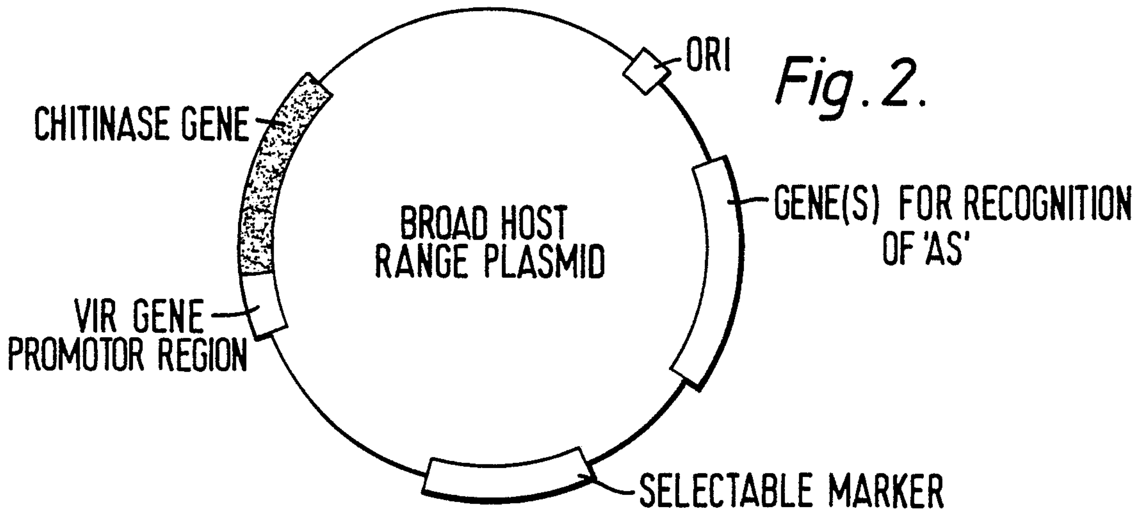


*Fig 3*

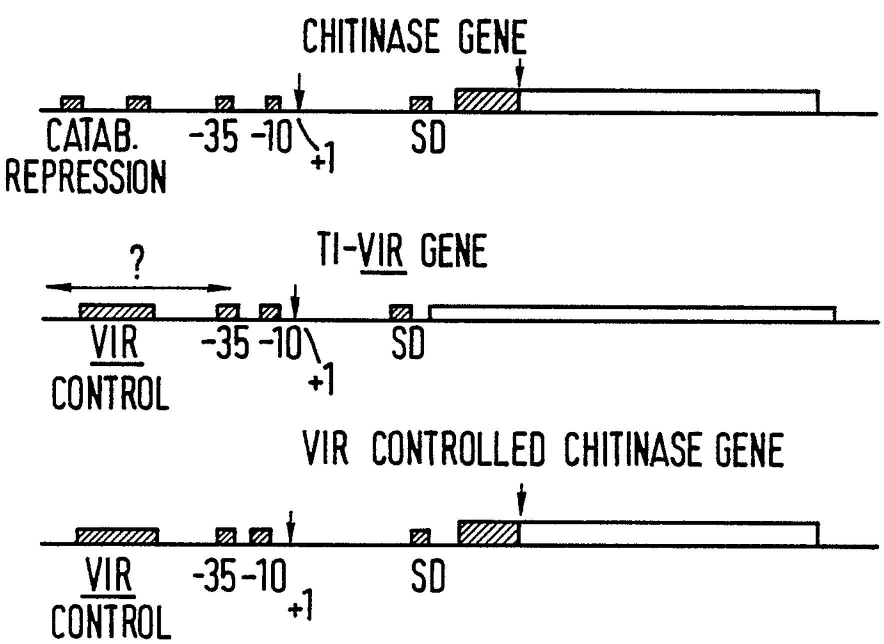

Fig. 4.

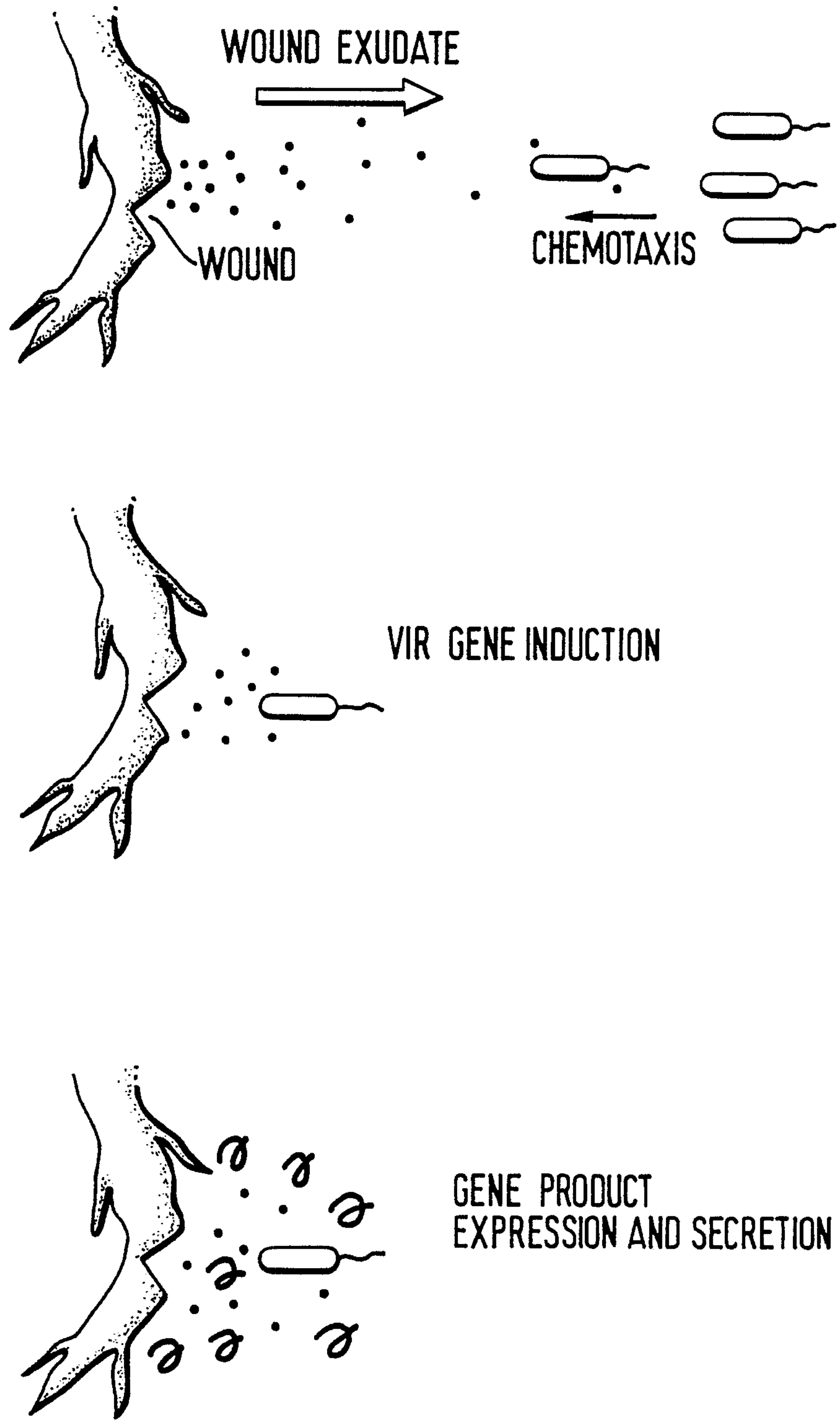
WOUND EXUDATE
WOUND
CHEMOTAXIS
VIR GENE INDUCTION
GENE PRODUCT
EXPRESSION AND SECRETION

Fig. 5. CHEMOTAXIS TOWARDS ACETOSYRINGONE

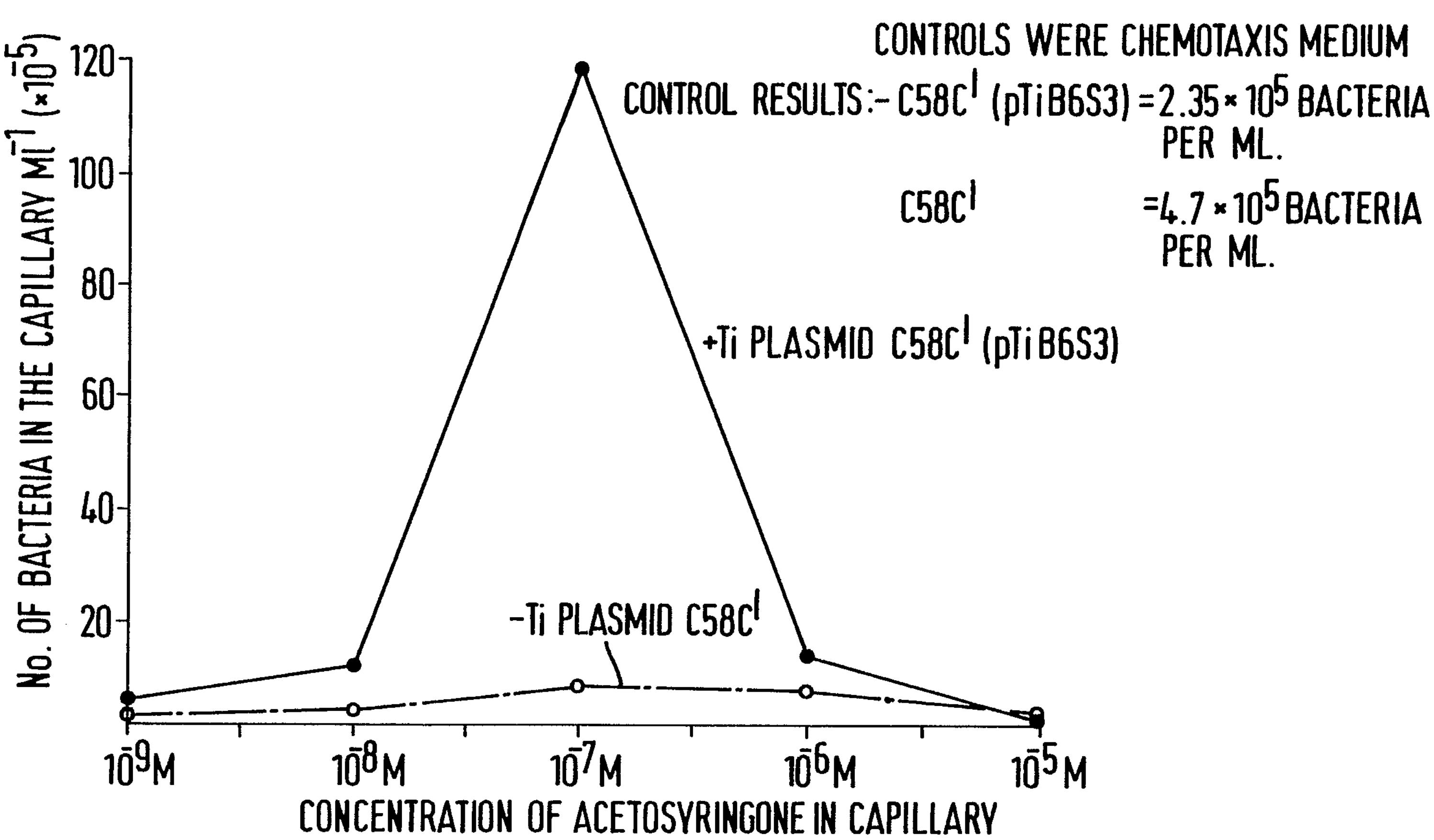

Fig. 6. CHEMOTAXIS TOWARDS VANILYLALCOHOL

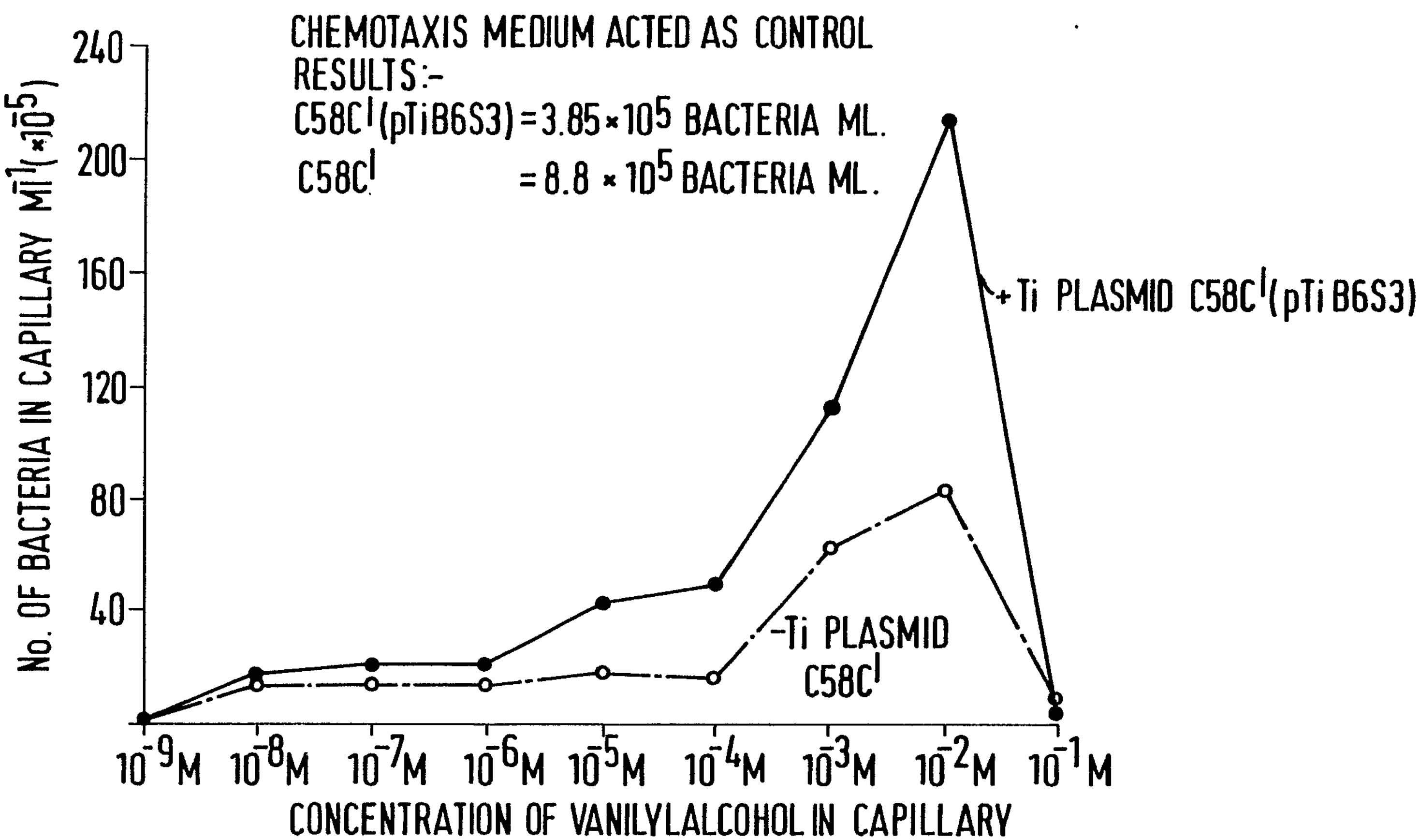

Fig. 7.

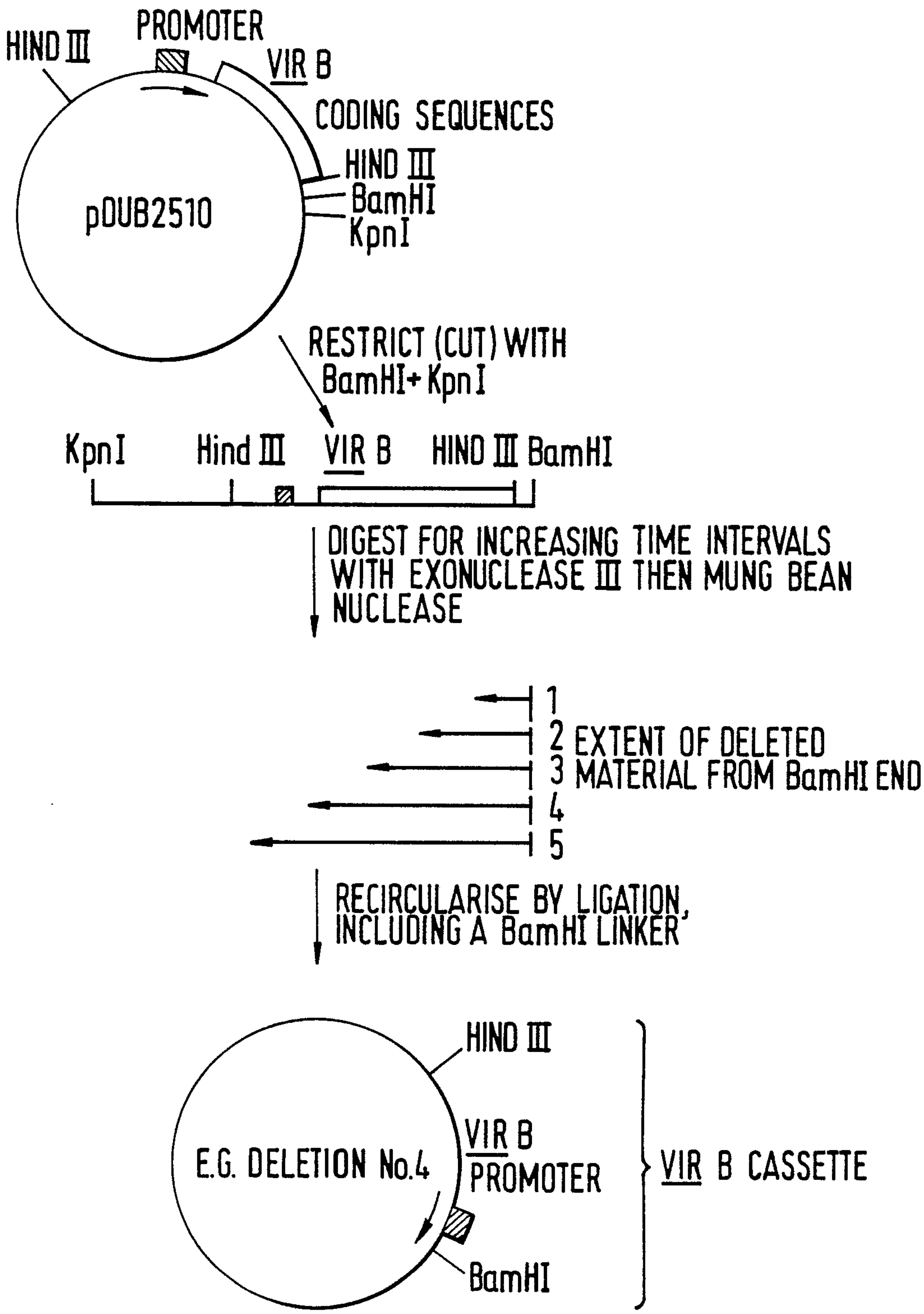
HIND III
PROMOTER
VIR B
CODING SEQUENCES
HIND III
BamHI
KpnI
pDUB2510
RESTRICT (CUT) WITH
BamHI+KpnI
KpnI
Hind III
VIR B
HIND III BamHI
DIGEST FOR INCREASING TIME INTERVALS
WITH EXONUCLEASE III THEN MUNG BEAN
NUCLEASE
1
2 EXTENT OF DELETED
3 MATERIAL FROM BamHI END
4
5
RECIRCULARISE BY LIGATION,
INCLUDING A BamHI LINKER
HIND III
E.G. DELETION No.4
VIR B
PROMOTER
BamHI
VIR B CASSETTE

Fig.8.

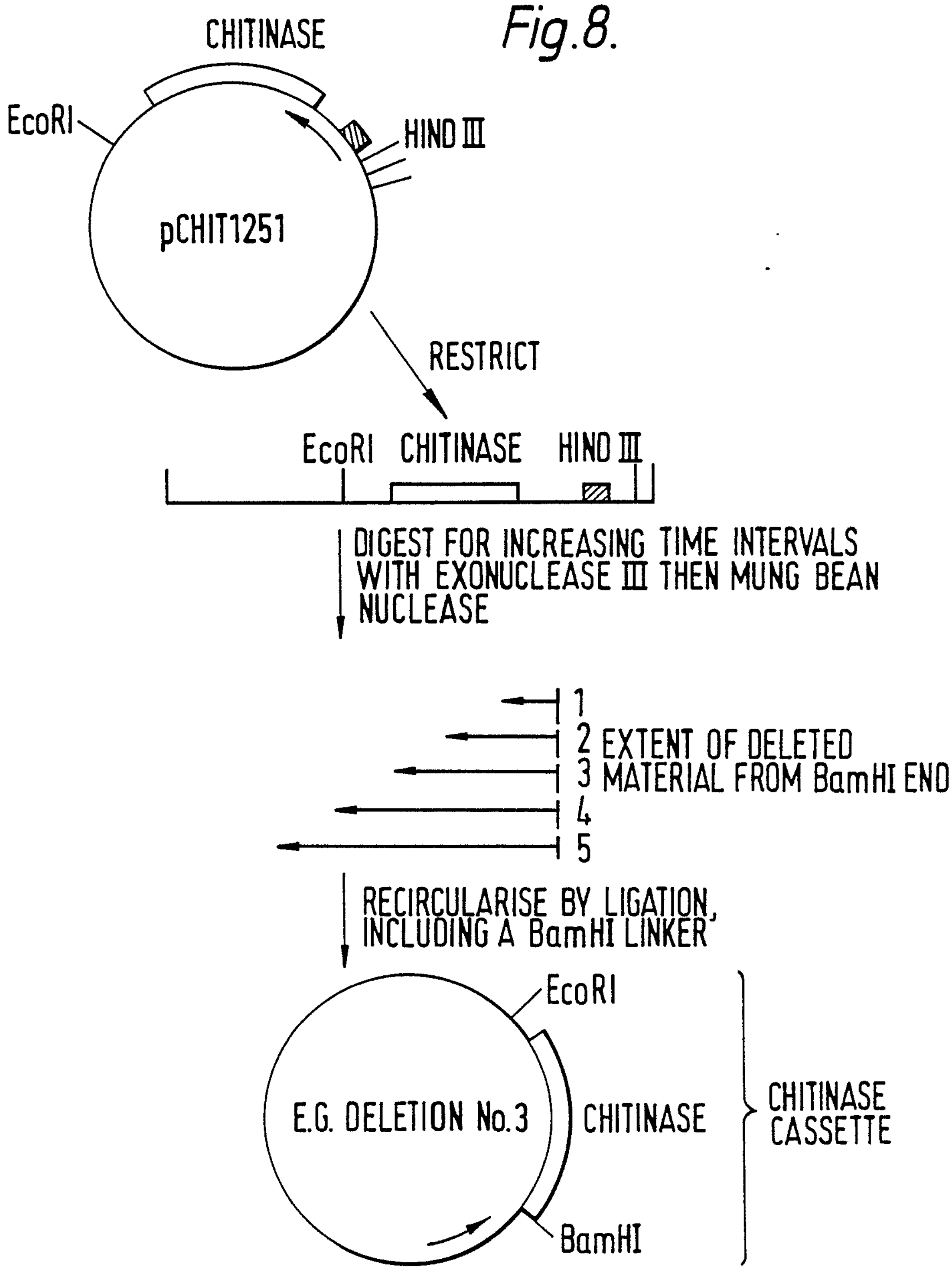
CHITINASE
EcoRI
HIND III
pCHIT1251
RESTRICT
EcoRI
CHITINASE
HIND III
DIGEST FOR INCREASING TIME INTERVALS WITH EXONUCLEASE III THEN MUNG BEAN NUCLEASE
1
2 EXTENT OF DELETED
3 MATERIAL FROM BamHI END
4
5
RECIRCULARISE BY LIGATION, INCLUDING A BamHI LINKER
EcoRI
E.G. DELETION No.3
CHITINASE
BamHI
CHITINASE CASSETTE

Fig. 9.

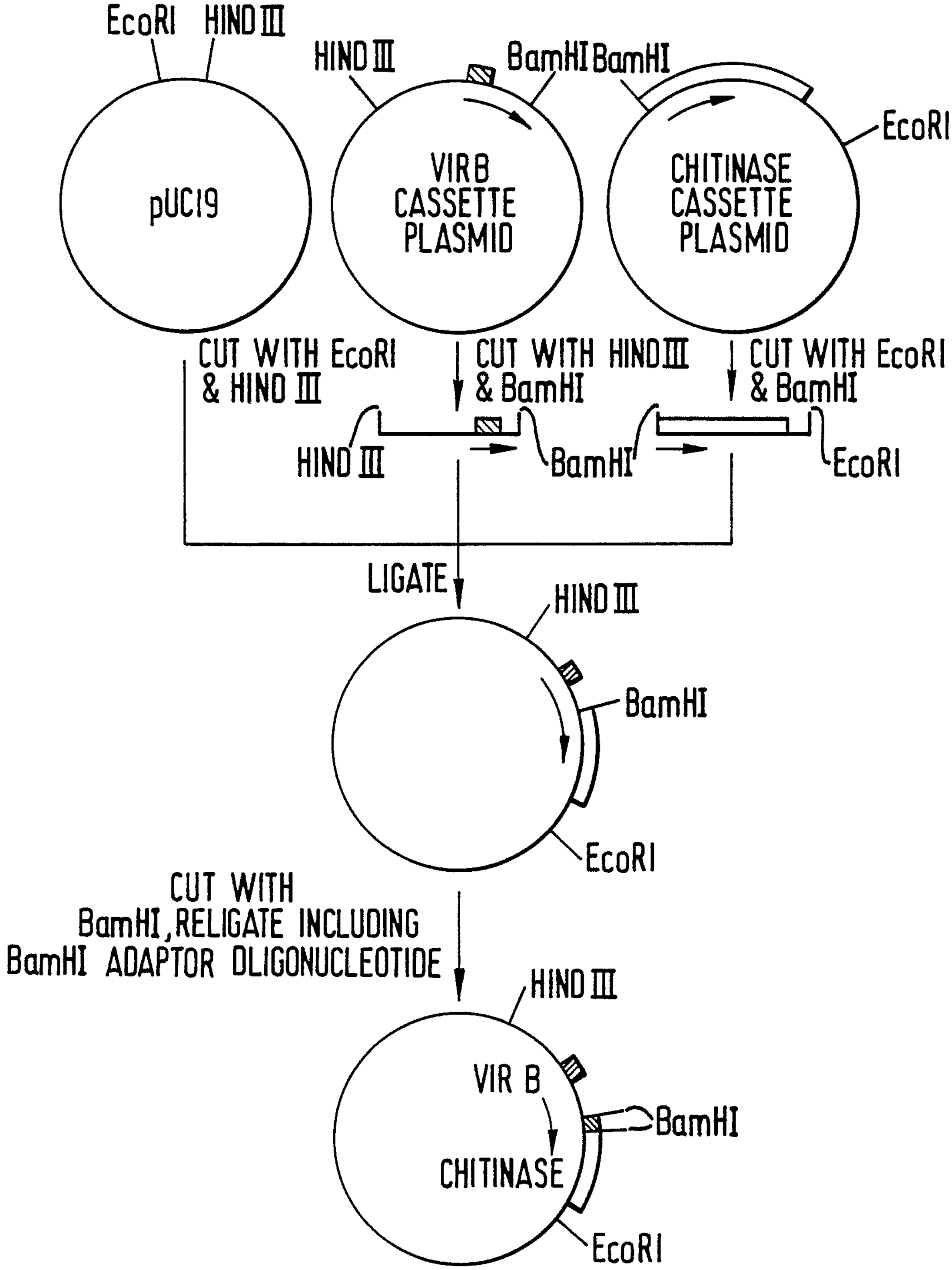
EcoRI
HIND III
pUC19
HIND III
BamHI
VIRB CASSETTE PLASMID
BamHI
EcoRI
CHITINASE CASSETTE PLASMID
CUT WITH EcoRI & HIND III
CUT WITH HINDIII & BamHI
CUT WITH EcoRI & BamHI
HIND III
BamHI
BamHI
EcoRI
LIGATE
HIND III
BamHI
EcoRI
CUT WITH BamHI, RELIGATE INCLUDING BamHI ADAPTOR OLIGONUCLEOTIDE
HIND III
VIR B
CHITINASE
BamHI
EcoRI

Fig. 10.

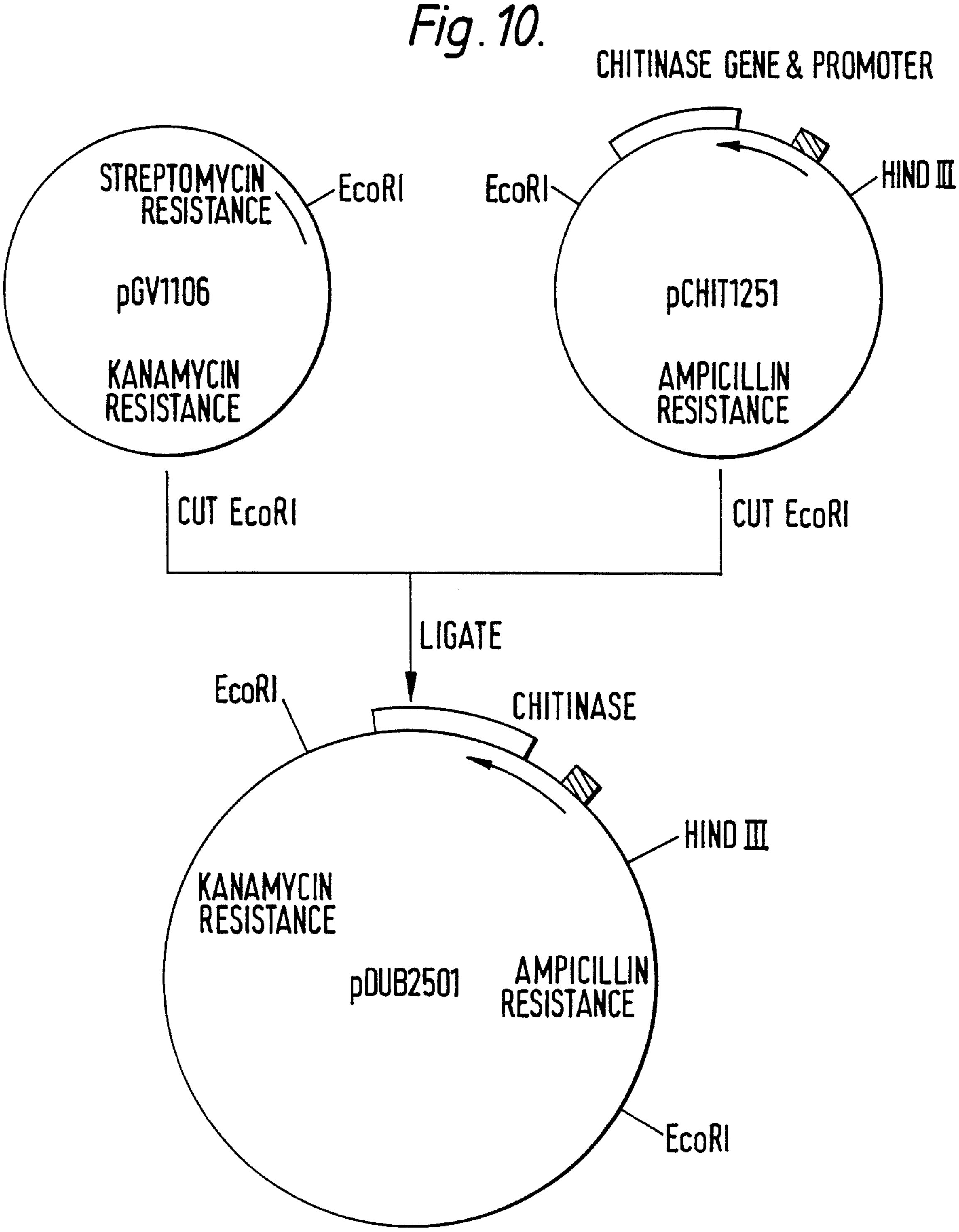
CHITINASE GENE & PROMOTER
STREPTOMYCIN RESISTANCE
EcoRI
pGV1106
KANAMYCIN RESISTANCE
EcoRI
HIND III
pCHIT1251
AMPICILLIN RESISTANCE
CUT EcoRI
CUT EcoRI
LIGATE
EcoRI
CHITINASE
HIND III
KANAMYCIN RESISTANCE
pDUB2501
AMPICILLIN RESISTANCE
EcoRI